# EUROPEAN PATENT SPECIFICATION

(11) **EP 2 355 691 B1**
(45) Date of publication and mention of the grant of the patent: **01.03.2017**
(21) Application number: 09745040.7
(22) Date of filing: 02.11.2009
(51) Int. Cl.: A61B 5/00

(54) **THERMOGRAPHIC PLATE FOR THE DETECTION OF TRACES OF BLOOD FLOW IN VESSELS AT DIFFERENT DEPTH**
THERMOGRAFISCHE PLATTE ZUR ERFASSUNG VON SPUREN DER DURCHBLUTUNG IN GEFÄSSEN IN UNTERSCHIEDLICHEN TIEFEN
PLAQUE THERMOGRAPHIQUE DESTINÉE À DÉTECTER DES TRACES DE FLUX SANGUIN DANS DES VAISSEAUX À DES PROFONDEURS DIFFÉRENTES

(30) Priority: 07.11.2008 IT BO20080673
(43) Date of publication of application: 17.08.2011
(73) Proprietor: LA3B S.r.l., 25067 Lumezzane (BS) (IT)
(72) Inventor: Montruccoli Salmi Daniele, deceased (IT)
(74) Representative: Pes, Matteo
(86) International application number: PCT/EP2009/064470
(87) International publication number: WO 2010/052193

(56) References cited:
- EP-A2- 0 858 770
- DE-A1- 19 927 426
- FR-A1- 2 551 892
- US-A- 5 183 031
- US-A- 5 995 865
- US-B1- 6 950 693

## Description

### Technical field

The present invention relates to a method for selective detection, with a thermographic plate, of traces of blood flow that differ from traces with correct course in vessels at different depths, particularly of the breast, and to an apparatus for performing the method and for generating thermographic maps of blood flow.

### Background Art

Apparatuses for thermographic investigation of the breast are known which provide the blowing of cold air onto the breast prior to placing thereon the thermographic plate in order to eliminate the influence of the surface heat of the mammary gland: this type of investigation seeks areas where an increase in heat occurs due to the presence of new blood vessels generated within the organ to supply a pretumoral or tumoral lesion: with this type of examination, a series of photographs, preferably of the digital type, of the thermographic plate placed with light pressure against the breast is taken, and the surface of the breast is cooled before each placement operation with a jet of air of a certain width, which strikes almost all of the mammary surface: the obtained images are viewed for identifying the presence, at different depths, of light-colored areolas (calcifications) that are denser, small and substantially circular (not elongated) in shape, which have been demonstrated to be indicators which are always present in precancerous or cancerous lesion situations.

US 5 995 865 discloses a thermographic device for examining blood circulation, comprising an elongated handle housing a small cooling fan therein, and an image aqcuisition camera and a frame for holding a thermographic plate provided at respective opposite ends of the handle.

EP 0 858 770 discloses a device for performing thermographic analysis of selected organ parts, comprising a hollow tube spacer at the opposite ends whereof there are rigidly coupled an imaging camera and a thermographic screen supporting frame. A fan is accommodated within the spacer and is suitable to cool the skin before resting the screen on the patient and taking at least one photograph.

As the investigated case series became longer, it has been found that with small modifications to the apparatus for thermographic investigation of the breast with cooling by blowing air it is possible to investigate the blood flow in breast vessels at different depths and to produce a map thereof without injecting contrast liquids and to identify, considerably in advance with respect to other investigation techniques, the traces of the vessels whose blood flow-rate differs from the norm and is an indicator of tumoral or pre-tumoral formations.

This is in agreement with the literature in the field, which teaches that for breast cancer, since there are no preventive measures or tests that can predict its onset, the only indicator to be sought for early diagnosis is observation of the onset of variations (increase) in blood inflow, such variations being necessary to supply the region where the tumor will appear, even before it is detectable as such.

### Disclosure of the Invention

The invention is defined by the appended claims. The aim of the present invention is to provide a method and an apparatus which, without the introduction of contrast liquids, allow respectively the selective detection of traces of breast blood circulation in vessels at different depths and performance of the method for generating blood flow maps.

Within this aim, an object of the present invention is to provide a method and an apparatus which are simple, relatively easy to provide in practice, safe to use, effective in operation, and have relatively low costs.

In accordance with the invention, there is provided a method for selective detection, with a thermographic plate, of traces of blood flow that differ from traces with correct course in vessels at different depths, particularly of the breast, as defined in the appended claims.

Also in accordance with the invention, there is provided an apparatus for performing the method for selective detection, with a thermographic plate, of traces of blood flow that differ from traces with correct course, also as defined in the appended claims.

### Brief description of the drawings

Further characteristics and advantages of the invention will become better apparent from the following detailed description of a preferred but not exclusive embodiment of a method for selective detection, with a thermographic plate, of traces of blood flow that differ from traces with correct course in vessels at different depths, particularly of the breast, and of an apparatus for performing the method and for generating thermographic maps of blood flow according to the invention, illustrated by way of non-limiting example in the accompanying drawings, wherein:
Figures 1a and 1b are schematic views of a method for selective detection, with a thermographic plate, of traces of blood flow that differ from traces with correct course in vessels at different depths, particularly of the breast;

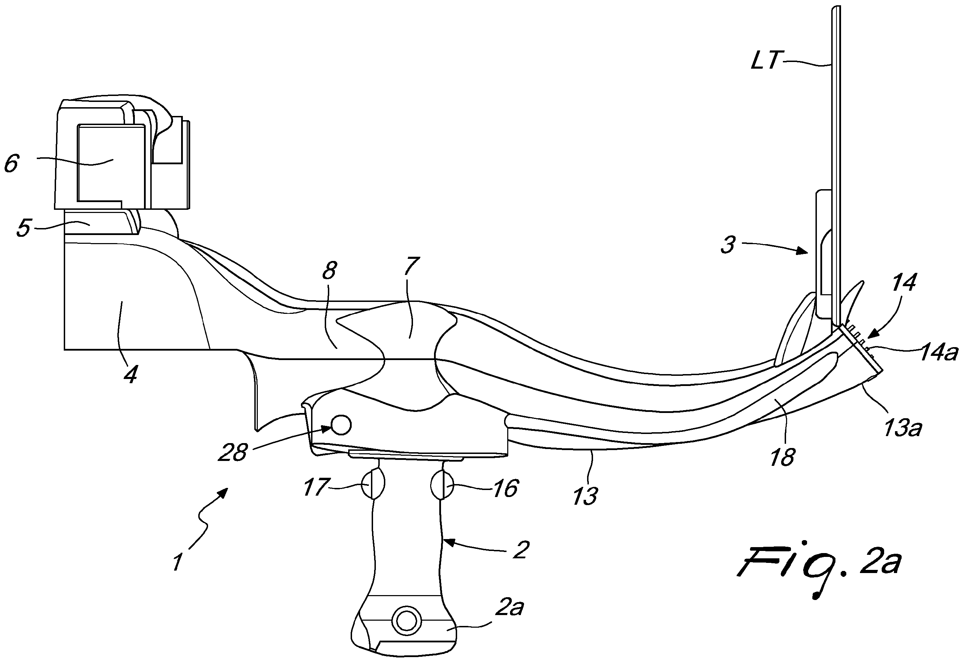

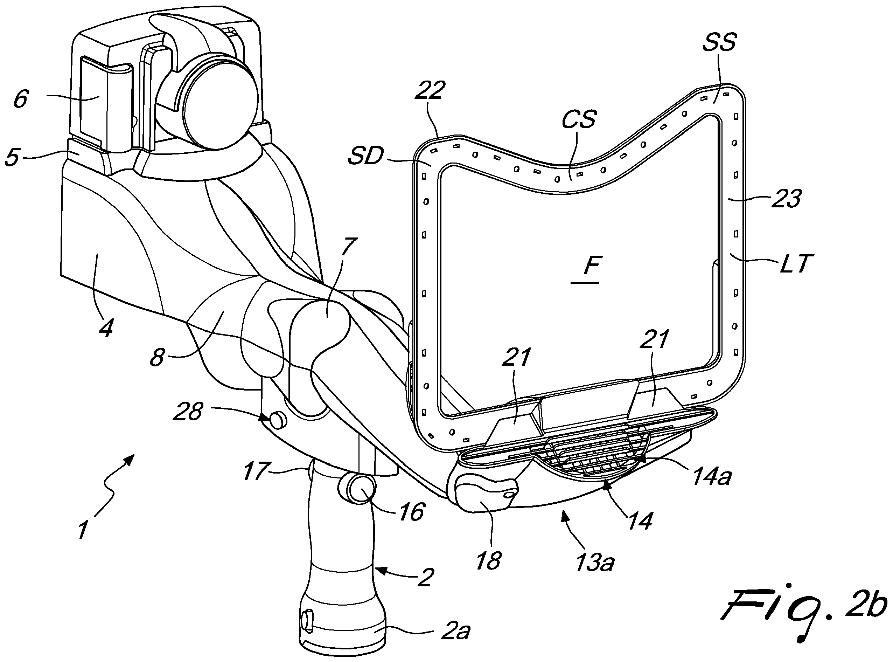

Figure 2a is a side view and Figure 2b is a front perspective view of an apparatus for performing the method and for generating thermographic maps of blood flow;

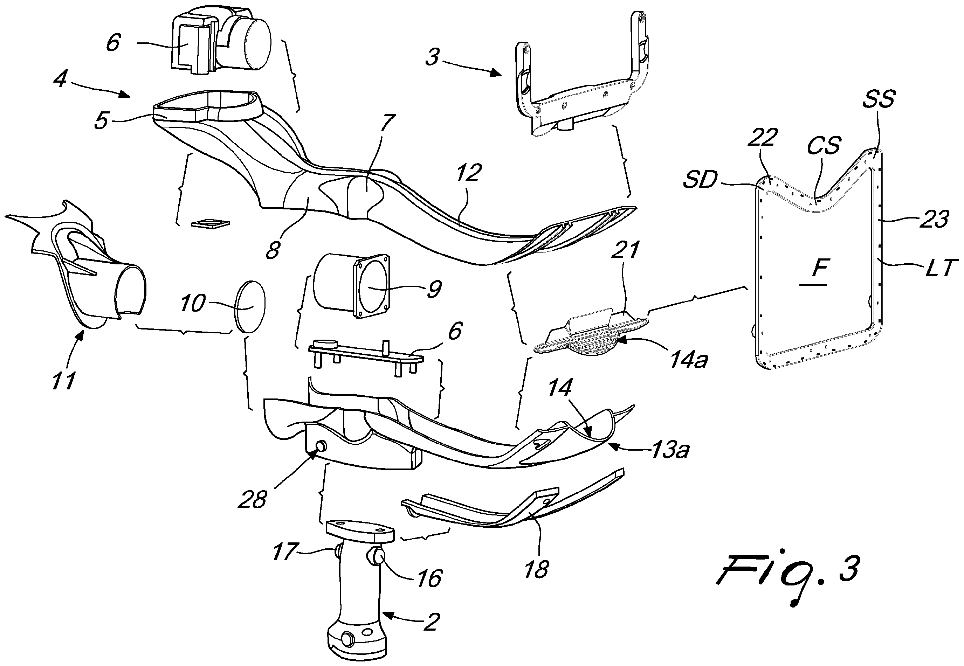

Figure 3 is an exploded perspective view of the apparatus according to the invention;

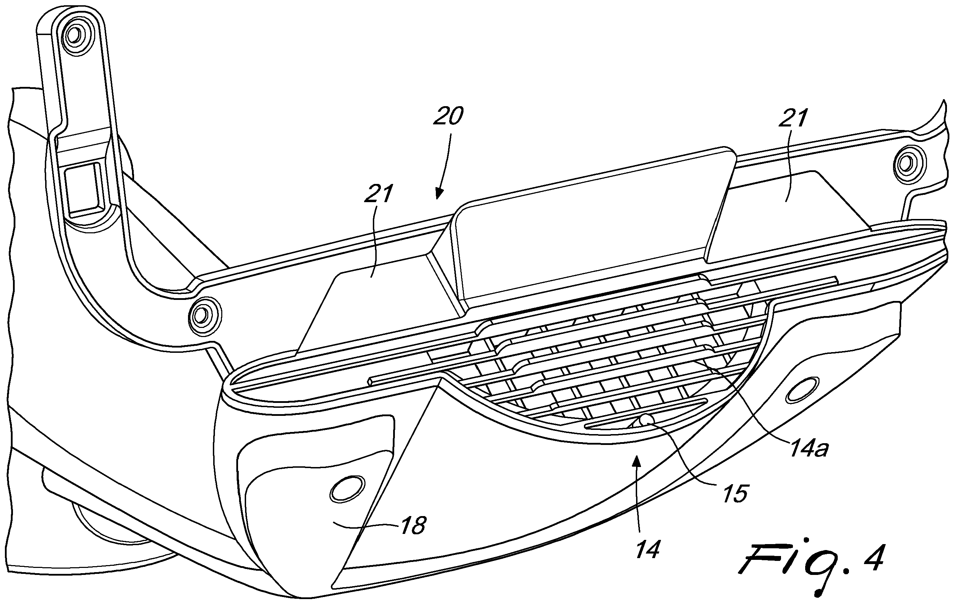

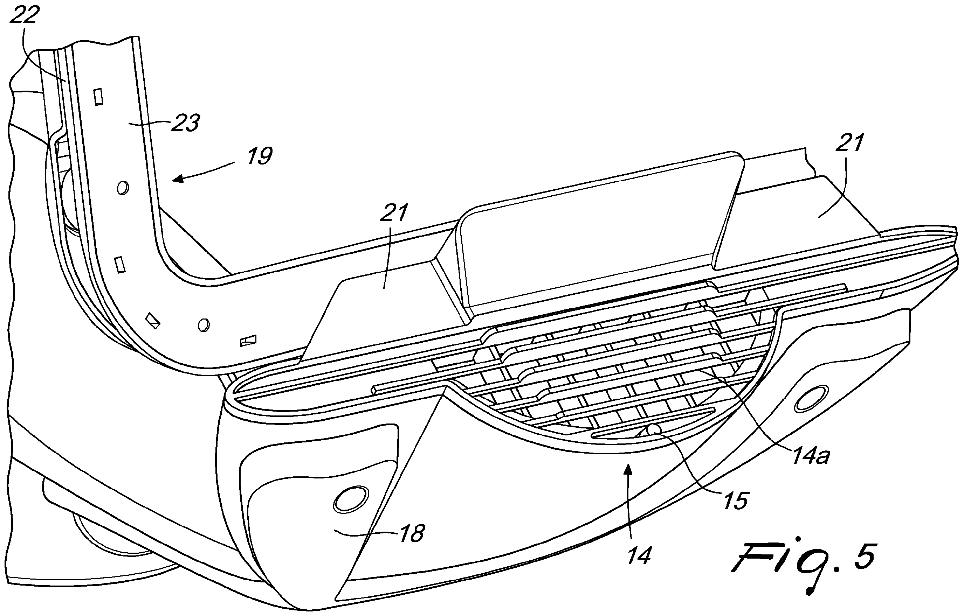

Figure 4 and Figure 5 are front perspective views of the front end of the apparatus according to the invention, respectively without and with the film supporting frame;

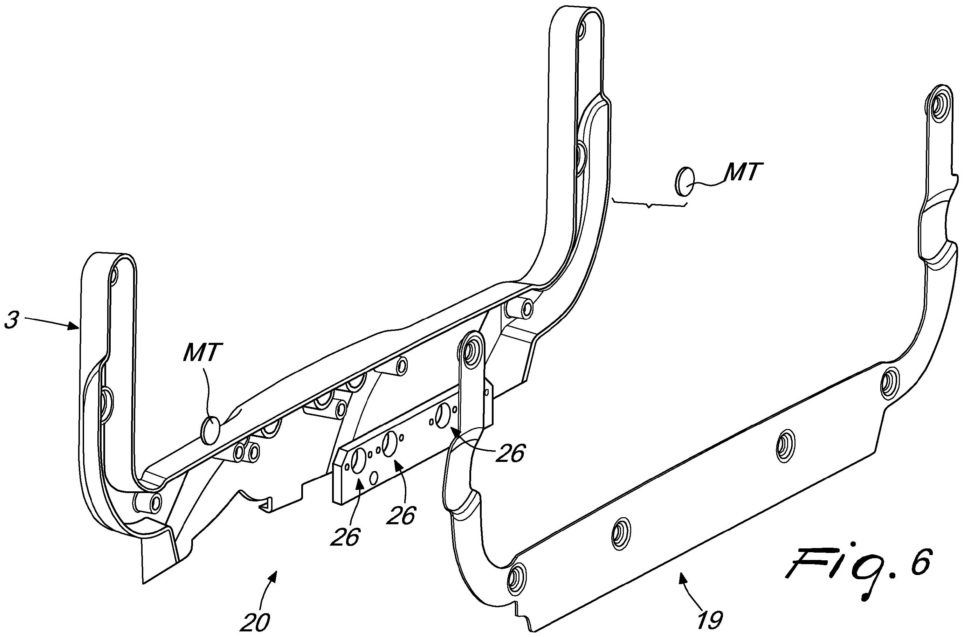

Figure 6 is an exploded front perspective view of the components of the frame for insertion of the thermographic plate;

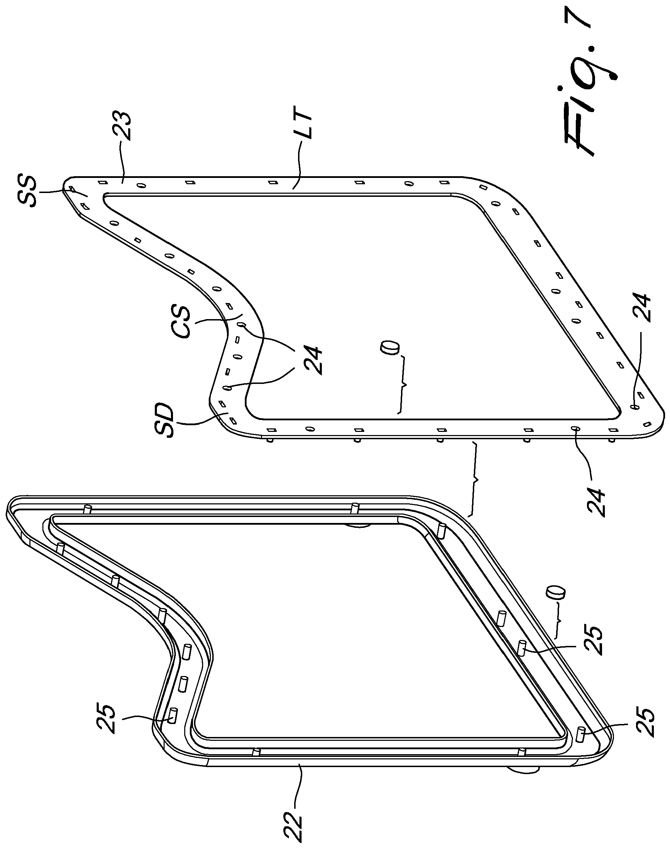

Figure 7 is an exploded perspective view of the frame of the thermographic plate.

### Ways of carrying out the Invention

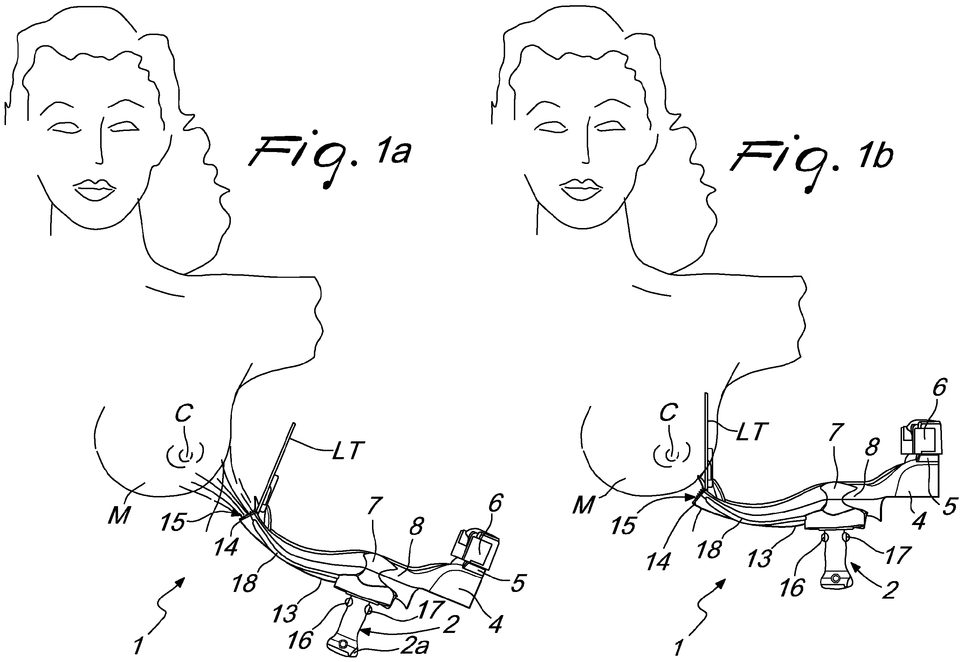

With reference to the figures, Figure 1 illustrates schematically a method for selective detection, with a thermographic plate LT, of traces of blood flow that differ from traces with correct course in vessels at different depths, particularly of the breast M: a sequence of thermographic images is taken, such images being acquired by the thermographic plate LT which is gently pressed against the skin, which, prior to taking of each image, is cooled with quick air jets whose duration is correlated to the depth of the portion of vessel being investigated; the traces of blood vessels with correct course have orientations which are substantially centripetal with respect to the breast and taper toward the center C of the organ to confirm a blood flow temperature that decreases because the vessel gradually branches, becoming thinner, and because the depth from the surface of the skin increases.

The apparatus for performing the method of Figure 1 comprises an elongated stand 1, which is hollow and contoured so as to convey longitudinally a stream of air, and is provided with a handle 2 and, at its two ends, with a frame 3 on the front for the snug insertion of a thermographic plate LT and on the rear with supporting means 4, which advantageously consist of a bracket 5 for resting the base of a camera MF and of a screw for coupling, by screwing in a threaded hole, the base of the camera: the base 2a of the handle is advantageously contoured and is provided with power supply laminae for coupling to the power supply of the batteries of the apparatus.

The stand 1 is provided by molding materials such as plastics and comprises an upper half-shell 7, which runs along the entire length of the stand and forms the bracket 5 on the rear, forms a box-like seat 8 in a substantially central position in which the concavity is directed downward for the placement of a support 6 for the electronic components and for the interlocking coupling of a tubular duct 9, in which an air blowing fan is accommodated with a corresponding electric motor and a filter 10, which are fitted in a lower cover for closing the rear end of the half-shell 7; in the front, the concave seat 8 of the half-shell 7 is flattened and forms a sort of wave 12, which is elongated longitudinally and has a flattened end section.

A lower half-shell 13 is fixed below the half-shell 7 and has, in the front region, a shape which is complementary to the wave 12 and forms, in cross-section, a central concavity 13a, which diverges upward and cooperates with the flat end section of the upper half-shell to form an outlet 14 for the outflow of the air jets which is central and concentrated, such outlet being located in front of the base and at the center of the frame and being protected by a grille 14a; the selective succession of the images taken after the respective blowing actions is considered independently of the colors assumed by the plate and as a function of the geometry of the traces, which are adapted to highlight the variation of the flow-rate of blood in the blood vessel.

The handle 2 is of the pistol type and is provided with a first trigger 16 for activating the motor of the fan and with a second trigger 17, which is operatively connected to the shutter release button of the camera: at the base of the air outflow duct there is a lamp 15, advantageously of the type known by the acronym LED, for lighting the area struck by the air stream: the lamp 15 also is activated by the first trigger 16.

The first and second triggers are located respectively at the front and at the rear on the top of the handle 2 to be actuated respectively with the index finger and with the thumb.

A contoured flat stiffening fork 18 is fixed below the front portion of the lower half-shell.

The frame 3 consists of two brackets 19 and 20, respectively on the front and on the rear, which are U-shaped and flattened and form, at their sides, two lateral clamps whose tips are drawn together by magnetic pellets 21 for coupling from above the two sides of the thermographic plate LT by sliding with snug fitting.

The plate LT is substantially rectangular and consists of a front frame 22 and a rear frame 23, which are provided with holes and pins 24, 25 for being fixed to each other and with the right upper corner SD and the left upper corner SS and the upper side LS which are curvilinear, respectively convex and concave, and allow access to locations of the body, for example the armpit, which would not be accessible with a straight upper side and with sharp upper corners.

The method according to the invention comprises in succession the step of activating the fan motor to blow quick jets of air against the skin region, the step of resting the thermographic plate against the skin region, the step of taking at least one photograph of the thermographic plate with the camera and the step of sorting the photographic images so that they represent, by means of groups of successive images, blood flow in the various vessels.

The thermographic plate comprises a thin support, which is stretched between the two frames 22, 23, has a thickness comprised between two and eight hundredths of a millimeter and is advantageously made of cellophane or of a material known by the trade-name Mylar.

At the base and at the center of the frame 3, in the portion of the bracket 20 that is directed toward the camera, it is possible to arrange in a horizontal row abbreviated codes 26, which can be selected among a series of indicator disks, which indicate at a glance the type of photograph, the body part involved, and other information related to the photograph; as an alternative, the codes can be selected and listed by means of a button 28, which is located above the pistol-grip handle, in a preset position of the photograph.

At the base of the bracket 19 there is a tab 27, which forms a pocket for the insertion of the base of the frame of the plate LT from above.

The plates used in the past were of a rather flexible, deformable and heat-sensitive type, whereas the ones currently used are more rigid.

The operation of the invention is relatively simple. By means of successive quick air jets, the skin of the breast is cooled and the thermographic plate is rested against the breast, pressing gently; the lines of the blood vessels revealed in a healthy breast comprise elongated traces of a certain width, which are directed substantially centripetally with respect to the breast and toward the center of the breast taper slightly and form a tip which substantially indicates that the blood vessel being investigated has branched or that its distance from the surface of the skin has increased.

If truncated or ball-shaped or crescent-like traces, or traces that branch into two or three lines appear instead of the correct elongated and pointed traces, with images that persist even despite selective cooling, this is an indicator of the presence, in the breast, of regions with increased blood inflow.

Using additional quick air jets, it is possible to cool the tissues at greater depth and follow the blood flow in the main vessels in order to check that there is no sudden reduction in blood flow, a non-pointed vessel trace, indicating that in that specific region of the breast the tissues demand a greater supply of blood in a situation that normally precedes the formation of tumors.

A time marking device is advantageously installed in the frame and records the overall duration of the cooling jets and prints it on the thermographic plate in a preset position.

Blood flow traces may also occur in regions that do not correspond to the main vessels; such traces, which are not pointed, indicate the formation of new vessels suitable to supply tumoral lesions. Advantageously, two series of thermographic recordings are made, one with the thermographic plate that reacts at a temperature of 32°C and the other one at 33°C; the series conveniently consist of four views each, namely Right Lateral, Right Frontal, Left Frontal, Left Lateral; the Lateral/Frontal views and the different sensitivity of the plates are adapted to provide a spatial representation of the vessels.

The thermographic investigation according to the invention has proved to be effective when the investigation is performed on a patient for the first time; the investigation is far more effective if it is possible to compare it with a similar investigation performed earlier: in this case it is possible to note that in the absence of neotumoral formations, the blood flow in the various vessels remains the same, whereas even small variations in the traces of blood flow in some vessels indicate an increased blood supply activity for the tissues to be considered as alarming.

The method and the apparatus according to the invention have yielded extremely interesting results even in a young female population, under 40 years of age, a population for which known investigation systems are unable to report situations of concern.

With the method and the apparatus according to the invention it is possible to identify a lobular tumor, which is generally an occasional finding, since it is not involved in the formation of micro-calcifications or lacks of organization that are clearly visible with a mammogram: however, the lobular tumor, by having a microcirculation that is similar to that of the ductal tumor, can be identified easily with the method and the apparatus according to the invention.

It is noted that the method and the apparatus according to the invention work non-invasively, without emitting radiation and without the injection of contrast liquids: therefore, it is possible to perform the investigation whenever one wishes, even a short time after an investigation of any type.

It has thus been shown that the invention achieves the intended aim and objects.

The invention thus conceived is susceptible of numerous modifications and variations, all of which are within the scope of the appended claims.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and dimensions, may be any according to requirements without thereby abandoning the scope of the protection of the appended claims.

## Claims

1. An apparatus for performing a method for selective detection, with a thermographic plate, of traces of blood flow that differ from traces with a correct course in the blood vessels of a patient at different depths, particularly the vessels of a breast, comprising a stand (1) provided with a handle (2) which has, at its two ends, a frame (3) for the snug fit of a thermographic plate (LT) and supporting means (4) for a camera (MF), a cooling fan associated with said stand (1) for blowing a jet of air against a skin region of a patient, which is mounted and actuated so that it can rotate in a duct (9) whose outlet (14) is located in the front of the base and at the center of said frame (3), the selective succession of the images taken after respective air jets being considered independently of the colors assumed by the plate and as a function of the geometry of the traces, which are suitable to highlight the variation of the blood flow-rate in said vessel, and a lamp (15), **characterized in that** said lamp is an LED lamp (15) arranged at the base of the outlet (14) of the air outflow duct, said outlet (14) having a reduced area, said lamp (15) being actuatable by a first trigger (16) for activating the motor of said cooling fan for lighting the area of the skin region struck by the air stream, and **in that** it further comprises a time marking device that is installed in the frame (3) and records the overall duration of the cooling jets and prints it on the thermographic plate (LT).

2. The apparatus according to claim 1, **characterized in that** said handle (2) is of the pistol type and **in that** there is a first trigger (16) for activation of the motor that drives the fan and a second trigger (17) that is functionally connected to the shutter release button of said camera (MF).

3. The apparatus according to claim 2, **characterized in that** said first and second triggers (16,17) are located respectively on the front and on the rear at the top of said handle (2) to be operated respectively with the index finger and with the thumb.

4. The apparatus according to claim 1, **characterized in that** said thermographic plate (LT) has a substantially rectangular shape, in which the upper comers (SD,SS) and the upper side (LS) are curvilinear and respectively convex and concave and are adapted to allow access to particular locations of the body that are problematic to access, for example the armpit.

5. The apparatus according to one or more of the preceding claims, **characterized in that** said thermographic plate (LT) comprises a thin support, whose thickness is comprised between two and eight hundredths of a millimeter.

6. The apparatus according to one or more of the preceding claims, **characterized in that** said thin support of said thermographic plate (LT) is made of cellophane or of a material known by the trade-name Mylar.

7. The apparatus according to one or more of the preceding claims, **characterized in that** at the base of said frame (3) there are, in a horizontal row, abbreviated codes (26) which can be selected among a series of indicator disks, which are adapted to indicate at a glance information related to the photograph.

8. A method for using the apparatus according to one or more of the preceding claims for selective detection, with said thermographic plate (LT), of traces of blood flow that differ from traces with correct course in the blood vessels of a patient at different depths, particularly of the breast of a patient, wherein a sequence of thermographic images is taken which are acquired by said thermographic plate (LT) gently pressed against the skin, which is cooled before each photograph with quick air jets, the traces of vessels with correct course having a substantially centripetal direction with respect to the breast (M) and tapering toward the center (C) of the organ so as to confirm a temperature of the blood flow which decreases because the vessel gradually branches and tapers and because the depth from the surface of the skin increases, the method being **characterized in that** it comprises correlating the duration of the air jets to the depth of the portion of vessel being investigated, lighting the area struck by the air stream by means of said LED lamp (15) and recording the overall duration of the cooling jets by means of said time marking device and printing it on the thermographic plate (LT).

9. The method according to claim 8, **characterized in that** it comprises in succession the steps of: activating said fan motor to blow a jet of air against said skin region; resting said thermographic plate (LT) against said skin region; taking at least one photograph of said thermographic plate (LT) with said camera (MF); and sorting the photographs so as to represent with groups of successive images the blood flow in the various vessels.

10. The method according to claim 9, **characterized in that** two series of thermographic recordings are made, one with a thermographic plate (LT) that reacts at a temperature of 32°C and one at 33°C, presenting four views each, namely Right Lateral, Right Frontal, Left Frontal, Left Lateral, the Lateral/Frontal views and the different sensitivities of the plates (LT) being suitable to provide a spatial representation of the vessels.

## Patentansprüche

1. Vorrichtung zum Ausführen eines Verfahrens zum selektiven Nachweis, mit einer thermographischen Platte, von Spuren von Blutfluss, die sich von Spuren mit einem korrekten Verlauf in den Blutgefäßen eines Patienten in verschiedenen Tiefen unterscheiden, insbesondere den Gefäßen einer Brust, umfassend einen mit einem Handgriff (2) versehenen Ständer (1), der an seinen beiden Enden einen Rahmen (3) für den Passsitz einer thermographischen Platte (LT) und Tragmittel (4) für eine Kamera (MF) aufweist, wobei zum Blasen eines Luftstroms auf einen Hautbereich eines Patienten ein Kühlgebläse mit dem Ständer (1) verbunden ist, welches derart montiert ist und betätigt wird, dass es in einem Kanal (9) drehen kann, dessen Auslass (14) sich in der Vorderseite der Basis und in der Mitte des Rahmens (3) befindet, wobei die selektive Aufeinanderfolge der nach jeweiligen Luftstrahlen aufgenommenen Bilder unabhängig von den von der Platte angenommenen Farben und als Funktion der Geometrie der Spuren angesehen wird, und die geeignet sind, die Variation der Blutflussrate in dem Gefäß aufzuzeigen, und eine Lampe (15),
**dadurch gekennzeichnet, dass** die Lampe eine LED-Lampe (15) ist, die an der Basis des Auslasses (14) des Luftauslasskanals angeordnet ist, wobei der Auslass (14) eine reduzierte Fläche aufweist, wobei die Lampe (15) durch einen ersten Abzug (16) zum Aktivieren des Motors des Kühlgebläses zum Beleuchten der Fläche des durch den Luftstrom beaufschlagten Hautbereichs betätigbar ist, und dass sie ferner eine Zeitmarkierungsvorrichtung umfasst, die in dem Rahmen (3) installiert ist und die Gesamtdauer der Kühlströme registriert und sie auf die thermographische Platte (LT) druckt.

2. Vorrichtung nach Anspruch 1, **dadurch gekennzeichnet, dass** der Handgriff (2) vom Pistolentyp ist und dass ein erster Abzug (16) für die Aktivierung des Motors, der das Gebläse versorgt, vorgesehen ist, und ein zweiter Abzug (17) vorgesehen ist, der funktional mit dem Auslöseknopf der Kamera (MF) verbunden ist.

3. Vorrichtung nach Anspruch 2, **dadurch gekennzeichnet, dass** der erste und der zweite Abzug (16, 17) sich jeweils vorne und hinten am oberen Teil des Handgriffs (2) befinden, um jeweils mit dem Zeigefinger und dem Daumen bedient zu werden.

4. Vorrichtung nach Anspruch 1, **dadurch gekennzeichnet, dass** die thermographische Platte (LT) eine im Wesentlichen rechtwinklige Form aufweist, in der die oberen Ecken (SD, SS) und die obere Seite (LS) einen gekrümmten Verlauf aufweisen und konvex bzw. konkav und ausgebildet sind, Zugang zu bestimmten Stellen des Körpers zu erlauben, die problematisch zu erreichen sind, zum Beispiel die Achselhöhle.

5. Vorrichtung nach einem oder mehreren der vorhergehenden Ansprüche, **dadurch gekennzeichnet, dass** die thermographische Platte (LT) einen dünnen Träger umfasst, dessen Dicke zwischen zwei und acht Hundertsteln eines Millimeters beträgt.

6. Vorrichtung nach einem oder mehreren der vorhergehenden Ansprüche, **dadurch gekennzeichnet, dass** der dünne Träger der thermographischen Platte (LT) aus Cellophan oder einem unter dem Handelsnamen Mylar bekannten Material besteht.

7. Vorrichtung nach einem oder mehreren der vorhergehenden Ansprüche, **dadurch gekennzeichnet, dass** an der Basis des Rahmens (3) in einer horizontalen Reihe abgekürzte Codes (26) vorgesehen sind, die aus einer Reihe von Anzeigescheiben ausgewählt werden können, die ausgebildet sind, auf einen Blick Informationen über die Fotographie anzuzeigen.

8. Verfahren zur Verwendung der Vorrichtung nach einem oder mehreren der vorhergehenden Ansprüche für den selektiven Nachweis, mit der thermographischen Platte (LT), von Spuren von Blutfluss, die sich von Spuren mit einem korrekten Verlauf in den Blutgefäßen eines Patienten in verschiedenen Tiefen unterscheiden, insbesondere den Gefäßen einer Brust, wobei eine Folge von thermographischen Bildern aufgenommen wird, die von der vorsichtig gegen die Haut gedrückten thermographischen Platte (LT) erhalten werden, die vor jeder Fotographie mit schnellen Luftstrahlen gekühlt wird, wobei die Spuren der Gefäße mit korrektem Verlauf eine im Wesentlichen zentripetale Richtung mit Bezug auf die Brust (M) aufweisen und zur Mitte (C) des Organs konisch zulaufen, um so eine Temperatur des Blutflusses zu bestätigen, der abnimmt, da das Gefäß sich allmählich verzweigt und konisch zuläuft, und da die Tiefe von der Hautoberfläche her zunimmt, wobei das Verfahren **dadurch gekennzeichnet ist, dass** es das Korrelieren der Dauer der Luftstrahlen mit der Tiefe des untersuchten Gefäßteils, das Beleuchten des vom Luftstrom beaufschlagten Bereichs mittels der LED-Lampe (15) und das Registrieren der Gesamtdauer der Kühlströme mittels der Zeitmarkierungsvorrichtung und das Drucken derselben auf die thermographische Platte (LT) umfasst.

9. Verfahren nach Anspruch 8, **dadurch gekennzeichnet, dass** es in Aufeinanderfolge die folgenden Schritte umfasst: Aktivieren des Gebläsemotor zum Blasen eines Luftstroms auf den Hautbereich; Auflegen der thermographischen Platte (LT) auf den Hautbereich; Aufnehmen mindestens einer Fotographie der thermographischen Platte (LT) mit der Kamera (MF); und Sortieren der Fotographien, um so mit Gruppen von aufeinander folgenden Bildern den Blutfluss in den diversen Gefäßen darzustellen.

10. Verfahren nach Anspruch 9, **dadurch gekennzeichnet, dass** zwei Reihen von thermographischen Registrierungen vorgenommen werden, eine mit einer thermographischen Platte (LT), die bei einer Temperatur von 32 °C reagiert, und eine bei 33 °C, was jeweils vier Ansichten darstellt, nämlich rechts seitlich, rechts von vorn, links von vorn, links seitlich, wobei die Ansichten seitlich und von vorn und die verschiedenen Empfindlichkeiten der Platten (LT) geeignet sind, eine räumlich Darstellung der Gefäße zu liefern.

## Revendications

1. Appareil pour réaliser un procédé de détection sélective, avec une plaque thermographique, des traces de flux sanguin qui diffèrent de traces présentant une course correcte dans les vaisseaux sanguins d'un patient à des profondeurs différentes, en particulier les vaisseaux d'une poitrine, l'appareil comprenant un bâti (1) muni d'une poignée (2) qui présente, à ses deux extrémités, un cadre (3) pour l'ajustement serré d'une plaque thermographique (LT) et des moyens de support (4) pour un appareil photo (MF), un ventilateur de refroidissement associé avec ledit bâti (1) pour souffler un jet d'air contre une région de la peau d'un patient, le ventilateur étant monté et actionné de manière à pouvoir tourner dans un conduit (9) dont la sortie (14) est située devant la base et au niveau du centre dudit cadre (3), la succession sélective des images prises après les respectifs jets d'air étant considérée indépendamment des couleurs assumés par la plaque et en fonction de la géométrie des traces, qui sont aptes à mettre en évidence la variation du débit de sang dans ledit vaisseau, et une lampe (15), **caractérisé en ce que** ladite lampe est une lampe à LED (15) disposée au niveau de la base de la sortie (14) du conduit d'écoulement d'air, ladite sortie (14) ayant une aire réduite, ladite lampe (15) pouvant être actionnée par une première gâchette (16) pour activer le moteur dudit ventilateur de refroidissement pour illuminer la surface de la région de peau frappé par le flux d'air, et **en ce que** il comprend en outre un dispositif de marquage du temps qui est installé dans le cadre (3) et qui enregistre la durée totale des jets de refroidissement et l'imprime sur la plaque thermographique (LT).

2. Appareil selon la revendication 1, **caractérisé en ce que** ladite poignée (2) est du type pistolet, et **en ce que** il y a une première gâchette (16) pour l'activation du moteur entraînant le ventilateur et une deuxième gâchette (17) qui est fonctionnellement reliée au bouton de déclenchement de l'obturateur dudit appareil photo (MF).

3. Appareil selon la revendication 2, **caractérisé en ce que** lesdits première et deuxième gâchettes (16, 17) sont situées respectivement à l'avant et à l'arrière au niveau de la partie supérieure de ladite poignée (2) afin d'être actionnées respectivement avec l'index et le pouce.

4. Appareil selon la revendication 1, **caractérisé en ce que** ladite plaque thermographique (LT) présente une forme substantiellement rectangulaire, dont les coins supérieurs (SD, SS) et le côté supérieur (LS) sont curvilignes et, respectivement, convexe et concave, et sont adaptés pour permettre d'accéder à des emplacements particuliers du corps qui posent des problèmes d'accès, par exemple les aisselles.

5. Appareil selon une ou plusieurs des revendications précédentes, **caractérisé en ce que** ladite plaque thermographique (LT) comprend un support mince ayant une épaisseur comprise entre deux et huit centièmes de millimètre.

6. Appareil selon une ou plusieurs des revendications précédentes, **caractérisé en ce que** ledit support mince de ladite plaque thermographique (LT) est réalisé en cellophane ou en un matériau connu sous la dénomination commerciale Mylar.

7. Appareil selon une ou plusieurs des revendications précédentes, **caractérisé en ce que** des codes abrégés (26) se trouvent, dans une rangée horizontale, à la base dudit cadre (3) et peuvent être sélectionnés parmi une série des disques indicateurs qui sont adaptés pour indiquer, d'un coup d'oeil, une information relative à la photographie.

8. Procédé pour utiliser l'appareil selon une ou plusieurs des revendications précédentes pour la détection sélective, avec ladite plaque thermographique (LT), des traces de flux sanguin qui diffèrent de traces présentant une course correcte dans les vaisseaux sanguins d'un patient, dans lequel est prise une séquence d'images thermographiques qui sont acquises par ladite plaque thermographique (LT) doucement pressée contre la peau, qui est refroidie avant chaque photographie avec un jet d'air, les traces des vaisseaux avec une course correcte ayant une direction essentiellement centripète par rapport à la poitrine (M) et se rétrécissant vers le centre (C) de l'organe de façon à confirmer une température du flux sanguin qui diminue parce que le vaisseau progressivement se ramifie et se rétrécit et parce que la profondeur augmente à partir de la surface de la peau, le procédé étant **caractérisé en ce que** il comprend mettre en corrélation la durée des jets d'air avec la profondeur de la partie du vaisseau en cours d'étude, illuminer la surface frappé par le flux d'air au moyen de ladite lampe à LED (15) et enregistrer la durée totale des jets de refroidissement au moyen dudit dispositif de marquage du temps eL l'imprimer sur la plaque thermographique (LT).

9. Procédé selon la revendication 8, **caractérisé en ce que** il comprend successivement les étapes de: activer ledit moteur de ventilateur pour souffler un jet d'air contre ladite région de peau; appuyer ladite plaque thermographique (LT) contre ladite région de peau; prendre au moins une photo de ladite plaque thermographique (LT) avec ledit appareil photo (MF); et ranger les photos, de façon à représenter des groupes d'images successives du flux sanguin dans les différents vaisseaux.

10. Procédé selon la revendication 9, **caractérisé en ce que** deux séries d'enregistrements thermographiques sont réalisées, l'une avec une plaque thermographique (LT) qui réagit à une température de 32° C et l'autre à 33° C, chacune présentant quatre vues, c'est-à-dire latérale droite, frontale droite, frontale gauche, latérale gauche, les vues latérales/ frontales et les sensibilités différentes des plaques (LT) étant aptes à fournir une représentation spatiale des vaisseaux.
